Europäisches Patentamt

European Patent Office

Office européen des brevets

(11) Veröffentlichungsnummer: **0 282 474**
**A1**

## EUROPÄISCHE PATENTANMELDUNG

(21) Anmeldenummer: 88890048.7

(22) Anmeldetag: 10.03.88

(51) Int. Cl.⁴: **C 12 P· 7/06**
C 12 P 7/16, C 12 P 7/26

(30) Priorität: 10.03.87 AT 566/87

(43) Veröffentlichungstag der Anmeldung:
14.09.88 Patentblatt 88/37

(84) Benannte Vertragsstaaten: ES GR

(71) Anmelder: Effenberger, Helmut, Dipl.-Ing. Dr.
Florianigasse 38/13
A-1080 Wien (AT)

(72) Erfinder: Effenberger, Helmut, Dipl.-Ing. Dr.
Florianigasse 38/13
A-1080 Wien (AT)

Schmidt, Alfred, Prof. Dipl.-Ing. Dr.
Pacassistrasse 29
A-1130 Wien (AT)

Steiner, Eva, Dr.
Kleinengersdorferstrasse 8
A-2100 Korneuburg (AT)

Salzbrunn, Wolfgang, Dr.
Bräunlichgasse 11
A-2700 Wr. Neustadt (AT)

(74) Vertreter: Weinzinger, Arnulf, Dipl.-Ing. et al
Riemergasse 14
A-1010 Wien (AT)

(54) Verfahren zur kontinuierlichen Vergärung von kohlenhydrathältigen Medien mit Hilfe von Bakterien.

(57) Das Verfahren zur Vergärung von kohlenhydrathältigen Medien mit Hilfe von Butanol, Aceton, Ethanol und/oder Isopropanol bildenden Bakterien, wird wenigstens zweistufig ausgeführt, wobei im ersten Verfahrensabschnitt im wesentlichen die Bakterien kontinuierlich kultiviert und im zweiten kontinuierlich oder chargenweise geführten Verfahrensabschnitt im wesentlichen das Produkt gebildet wird.

Zwecks Erzielung einer Langzeitstabilität des Verfahrens und einer erhöhten Produktivität sowie einer erhöhten Zuckerabbaues werden die Bakterien im zweiten Verfahrensabschnitt an einem Trägermaterial immobilisiert. Dabi kann aus der Produktbildungsstufe Medium abgezogen und das gebildete Produkt abgetrennt werden, um eine Inhibierung durch zu hohe Produktkonzentraiton zu vermeiden.

In einer Vorrichtung zur Durchführung dieses Verfahrens sind ein Bioreaktor, vorzugsweise ein Rührkesselbioreaktor (4), und ein mit Mikroorganismen rückhaltendem Material bestückter Reaktor, vorzugsweise ein Festbettreaktor (7), hintereinandergeschaltet, wobei gegebenenfalls an den Festbettreaktor (7) eine externe Schlaufe zur, vorzugsweise kontinuierlichen, Produktabtrennung angeschlossen ist.

Fig. 1

EP 0 282 474 A1

**0 282 474**

### Beschreibung

Verfahren zur kontinuierlichen Vergärung von kohlenhydrathältigen Medien mit Hilfe von Bakterien

Die Erfindung bezieht sich auf ein Verfahren zur Vergärung von kohlenhydrathältigen Medien mit Hilfe von Bakterien, welche Butanol, Aceton, Ethanol und/oder Isopropanol bilden, wobei das Verfahren wenigstens zweistufig ausgeführt wird, wobei im ersten Verfahrensabschnitt im wesentlichen die Bakterien kontinuierlich kultiviert und im zweiten kontinuierlich oder chargenweise geführten Verfahrensabschnitt im wesentlichen das Produkt gebildet wird.

Bei einem bekannten Verfahren dieser Art sind zwei herkömmliche Züchtungsstufen vorgesehen, wobei man hit Hilfe der Durchflußrate bzw. mit Hilfe der Phosphatlimitierung den Verfahrensablauf so einstellt, daß in der ersten Stufe (Wachstumsstufe) die Bakterienkonzentration konstant hoch bleibt, d.h. daß sich ein Fließgleichgewicht (steady state) einstellt und daß bereits ein Teil des Substrats zu Butanol, Aceton und/oder Ethanol umgesetzt wird. In der zweiten Stufe soll dann eine möglichst hohe Ausbeute an den gewünschten Gärungsprodukten in möglichst kurzer Zeit erzielt werden. Wählt man in der ersten Stufe eine zu hohe Durchflußrate, so werden mehr Zellen ausgetragen als neue gebildet. Bei zu geringer Durchflußrate hingegen kann das Zellwachstum gegebenenfalls eingestellt werden.

Ein solches Verfahren hat den Nachteil, daß einerseits die erzielten Produktivitäten noch nicht befriedigend sind, und daß anderseits eine Langzeitstabilität der kontinuierlichen Kultur nicht gegeben ist, d.h. daß über einen längeren Zeitraum von hundert Stunden und mehr keine konstanten Produktivitäten erzielt werden können. Dies liegt darin, daß die gebildeten Produkte, nämlich Butanol, Aceton und/oder Ethanol bzw. Isopropanol für die Gärungsorganismen toxisch wirken, so daß bei einer gewissen Konzentration der gebildeten Produkte entweder die Produktbildung eingestellt oder aber die Bakterienkulturen erheblich geschädigt werden. Ein weiterer Nachteil des bekannten Verfahrens ist auch darin zu erblicken, daß halbwegs befriedigende Resultate nur mit Hexosen erzielbar sind.

Der Erfindung liegt die Aufgabe zugrunde, ein Verfahren der eingangs genanten Art zu schaffen, mit welchem über einen langen Zeitraum hindurch konstant günstige Produktivitätswerte erzielbar sind. Außerdem soll auf Grund des Verfahrens erzielt werden, daß auch Ausgangsmaterialien eingesetzt werden können, die nicht nur Hexosen beinhalten. Erfindungsgemäß wird diese Aufgabe dadurch gelöst, daß die Bakterien im zweiten Verfahrensabschnitt an einem Trägermaterial immobilisiert werden.

Bei einstufigen Verfahren ist es bereits bekannt, Bakterien zu immobilisieren, u.zw. werden bei diesem bekannten Verfahren Zellen in Ca-Alginat immobilisiert wobei Glucose als Substrat eingesetzt wird. Bei diesen bekannten Verfahren konnten die Produktivitäten zwar gesteigert werden, jedoch konnten auch hier über eine längere Gärungszeit, z.B. von mehreren Wochen, nur geringe Produktivitäten aufrechterhalten werden.

Das erfindungsgemäße Verfahren ist hinsichtlich der Durchflußrate sehr flexibel, da die lebensfähigen Bakterien in der zweiten Stufe durch Adhäsion am Trägermaterial festhaften, wobei die Durchflußrate im ersten Verfahrensabschnitt so auf den Verfahrensablauf abgestimmt werden kann, daß in diesem ersten Verfahrensabschnitt nahezu ausschließlich Bakterien gebildet werden, welche dann in die zweite Verfahrensstufe teilweise mitgenommen werden. In dieser zweiten Verfahrensstufe erhält man dann eine hohe Bakterienpopulation, da sich die lebensfähigen Bakterien an das Trägermaterial anheften, wogegen bereits abgestorbene Bakterienzellen mit dem produktangereicherten Medium ausgeschwemmt werden. Es kann also mit einer so hohen Durchflußrate in der ersten Stufe gearbeitet werden, daß die Konzentration an gebildeten Produkten nicht die Toxizitätsgrenze überschreitet, so daß die Bakterienkultur im wesentlichen nicht geschädigt wird. Vorteilhafterweise kann als Trägermaterial ein offenporiges Sinterglas oder Bimsstein oder ein gleichwertiges Material mit hohem $SiO_2$ Gehalt, offenporiger Struktur, einer Porengröße von 20 - 200µm, vorzugsweise 50 - 100µm und einer Wasseraufnahmefähigkeit von gleich oder größer 0,35 ml/cm$^3$ eingesetzt wird, und wobei das Verhältnis des Volumens des Trägermaterials zum Gesamtarbeitsvolumen zwischen 1:10 bis 1:1,1, vorzugsweise 1:5 bis 1:6 beträgt, wodurch eine besonders wirksame Vergärung erzielt wird, insbesondere wenn in dem ersten Verfahrensabschnitt unter Durchmischung des Reaktorinhalts eine Durchflußrate von 0,08 bis 0,45 x h$^{-1}$, insbesondere 0,1 - 0,38h$^{-1}$, ein pH von 4,0 - 5,5, insbesondere 4,3 bis 5,1 vorzugsweise 4,5 - 5 und/oder eine Temperatur von 30° - 40° insbesondere 33° - 38°C eingehalten werden, wobei im zweiten Verfahrensabschnitt eine Durchflußrate von 0,14 - 0,4 x h$^{-1}$, vorzugsweise 0,25 -0,35 h$^{-1}$, ein pH von 3,0 - 5,0, insbesondere 4,0 - 4,8 vorzugsweise 4,2 -4,5, und/oder eine Temperatur von 27° -40°C, insbesondere 30° - 37°C, vorzugsweise 32° - 35°C, eingehalten werden.

Bei eine weiterer vorteilhaften Ausgestaltung des erfindungsgemäßen Verfahrens kann aus der Produktbildungsstufe Medium abgezogen, das gebildete Produkte extraktiv, vorzugsweise in an sich bekannter Weise mittels höherer Alkohole oder höherer Fettsäuren, abgetrennt und die dadurch anfallende produktarme Phase in die Produktbildungsstufe und/oder den ersten Verfahrensabschnitt rückgeführt werden. Dadurch wird erreicht, daß im zweiten Verfahrensabschnitt die Produktkonzentration niedrig gehalten wird, wodurch eine Schädigung der Bakterien hintangehalten wird, so daß immer eine sehr hohe Produktbildungsrate aufrechterhalten wird, u.z.w. auch über einen sehr langen Zeitraum. Weiters wird ermöglicht, den Abtrennungsschritt auzuführen, ohne damit die eigentliche Gärstufe zu beeinträchtigen. Es kann jedoch im zweiten Verfahrensabschnitt Produkt, vorzugsweise kontinuierlich, pervaporativ aus dem Medium abgeführt werden. Dabei kann für die pervaporative Produktentnahme, eine Pervaporationsmembran, insbesonders aus Silikonkautschuk, eingesetzt werden, wobei eine Temperatur von 30° - 90°C, vorzugsweise 40° - 80°C, eingehalten wird und wobei die Dampfdruckerniedrigung pervoporationsseitig durch ein Trägergas, wie z.B. $N_2$, $H_2$, $CO_2$ oder Mischungen dieser Gase oder auch ein Fermentationsgas bzw. durch Anlegen eines Vakuums erzielt wird. Es kann jedoch im zweiten Verfahrensabschnitt eine Produktabtrennung, vorzugsweise kontinuierlich, auch mittels Gasstrippen durchgeführt werden. Dabei kann für die kontinuierliche Produktabtrennung durch Gasstrippen als Stripgas z.B. $N_2$, $H_2$, $CO_2$ oder Mischung dieser Gase bzw. Fermentationsgas eingesetzt werden, wobei das Verhältnis der Volumenströme von Flüssigkeit zu Gas 0,05:1 bis 1,6:1 und die Temperatur 30° - 90°C, vorzugsweise 40° - 80°C, betragen.

All die angeführten Verfahren zur Produktabtrennung bewirken neben der Einsetzbarkeit von unkonventionellen Zuckern eine höhere Produktivität, die Möglichkeit eines Einsatzes von höher konzentrierten Zuckerlösungen und eine hohe Langzeitstabilität.

Für eine besonders genaue Regelung des Verfahrensablaufes kann der Zulauf an Nährlösung und/oder Zuckerlösung in Abhängigkeit vom Produktgehalt des zweiten Verfahrensabschnittes geregelt werden. Dadurch lassen sich besonders genau jene Konzentrationen an Nährlösung bzw. Zucker einstellen, die für einen optimalen Verfahrensablauf erforderlich sind.

Bei einer erfindungsgemäßen Vorrichtung zur Durchführung des erfindungsgemäßen Verfahrens kann ein Bioraktor, vorzugsweise ein Rührkesselbio reaktor, und ein mit Mikroorganismen rückhaltendem Material bestückter Reaktor, vorzugsweise ein Festbettreaktor, hintereinandergeschaltet sein, wobei an dem Festbettreaktor eine externe Schlaufe zur, vorzugsweise kontinuierlichen, Produktabtrennung angeschlossen ist. Durch eine solche Reaktorkombination läßt sich auf besonders einfache Weise einerseits die Wachstumsgeschwindigeit der eingesetzten Mikrooganismen und anderseits die Produktbildung in der zweiten Verfahrensstufe regeln.

Dabei können innerhalb des Festbettreaktors ein oder mehrere Leiteinrichtungen zur Erzielung einer Zirkulation innerhalb des Reaktors vorgesehen sein. Dadurch wird erreicht, daß innerhalb des Reaktors an allen Stellen etwa gleiche Konzentrationen an Nährsubstrat und auch an Produkt vorhersschen. Dabei können zur Erzielung der Zirkulation örtlich Gaseinbringungseinrichtungen oder Leitungen zum gerichteten Einströmen von, vorzugsweise rückgeführtem, Medium vorgesehen sein. Im Falle von Gaseinbringung wird dabei die Zirkulation durch eine Mammutpumpenwirkung erreicht, wogegen bei gerichtetem Einströmen von Flüssigkeit die Zirkulation durch die entsprechende Flüssigkeitsgeschwindigkeit erreicht wird.

In der externen Schlaufe kann zur Produktabtrennung ein Pervaporationsmodul angeordnet sein. Bevorzugt kann in dem Pervaporationsmodul eine Membran, insbesondere eine Silikonkautschukmembran, vorgesehen sein. Dadurch wird eine besonders günstige Abtrennung des Produktes aus dem Medium erzielt, wobei eventuell durch die Anströmung der Membran im Querstrom ein Ablagern von Mikroorganismen an der Membran verhindert wird. Schließlich kann in der externen Schlaufe zur Produktabtrennung eine Gegenstromstrippkolonne angeordnet sein. Es hängt dabei vom eingesetzten Ausgangsmaterial, von den eingesetzten Organismen und auch vom gebildeten Produkt ab, welche der Möglichkeiten zur Produktabtrennung herangezogen werden.

Zusammenfassend ist zu bemerken, daß in der ersten Verfahrensstufe eine derartig hohe Durchflußrate gewählt wird, daß sich die Bakterien in der Wachstumsphase und Säurebildungsphase befinden, ohne daß eine nennenswerte Produktbildung stattfindet. Die Bakterien entwickeln in dieser Wachstumsphase gute Adhäsionseigenschaften, die bei ihrem Übergang in den zweiten Verfahrensabschnitt dann zur Immobilisierung der Bakterien am Trägermaterial führen. Wird im ersten Verfahrensabschnitt seine zu geringe Durchflußrate gewählt, so wird das Zellwachstum, gegebenenfalls unter gleichzeitiger Produktbildung, eingestellt, und damit gehen auch die guten Adsorptionseigenschaften der Bakterien gegenüber dem Trägermaterial verloren. Anstelle einer Immobilisierung könnten im zweiten Verfahrensabschnitt die Zellen mittels Mikrofiltration, vorzugsweise cross-flow Mikrofiltration aus dem Medium abgetrennt und rückgeführt werden, wodurch die gleiche Wirkung wie durch Immobilisierung erzielt wird.

Zusätzlich führt eine hohe Produktkonzentration in der ersten Stufe (bei niedriger Durchflußrate) zu einer Degenerierung der Bakterien, was zu einem Wechsal von der Produktbildung zur erneuten Säurenbildung führt, welcher Wechsel erfahrungsgemäß nicht mehr rückgängig gemacht werden kann. Bei zu hoher Durchflußrate in der ersten Stufe kommt es anderseits zu einem Auswaschen der Bakterien, so daß nach einiger Zeit nicht mehr genügend Bakterien für den zweiten Verfahrensabschnitt nachgeliefert werden.

Es ist also die Durchflußrate entsprechend den jeweiligen Gegebenheiten einzustellen.

In der Zeichnung sind schematisch verschiedene Ausführungsbeispiele der erfindungsgemäßen Vorrichtung dargestellt. Fig. 1 zeigt das Verfahrensschema, mit welchem das gebildete Produkte durch Pervaporation abgeschieden wird. Fig. 2 zeigt das Verfahrensscheme, bei welchem die Produktabtrennung dur Extraktion erfolgt. Fig. 3 gibt das Verfahrensschema wieder, bei welchem das Produkt durch Strippen aus dem Medium ausgetrieben wird.

Bei allen drei Ausführungsbeispielen sind die gleichen Teile mit gleichen Bezugszeichen versehen. Mit 1 ist ein Vorratsgefäß bezeichnet, von welchem über eine Leitung 2 mittels einer Pumpe 3 die Nährlösung in den ersten Verfahrensabschnitt, welcher durch einen Rührkesselbioreaktor 4 gebildet ist, eingebracht wird. Von dem Rührkesselbioreaktor wird das mit Biomasse angereicherte Nährsubstrat über eine Leitung 5 und eine Pumpe 6 der zweiten Verfahrensstufe zugeführt, welche durch eine Festbettreaktor 7 gebildet ist. Von diesem Festbettreaktor geht eine Leitung 8 aus, über welche mittels einer Pumpe 9 das an Produkt angereicherte Medium aus dem Festbettreaktor abgeführt wird.

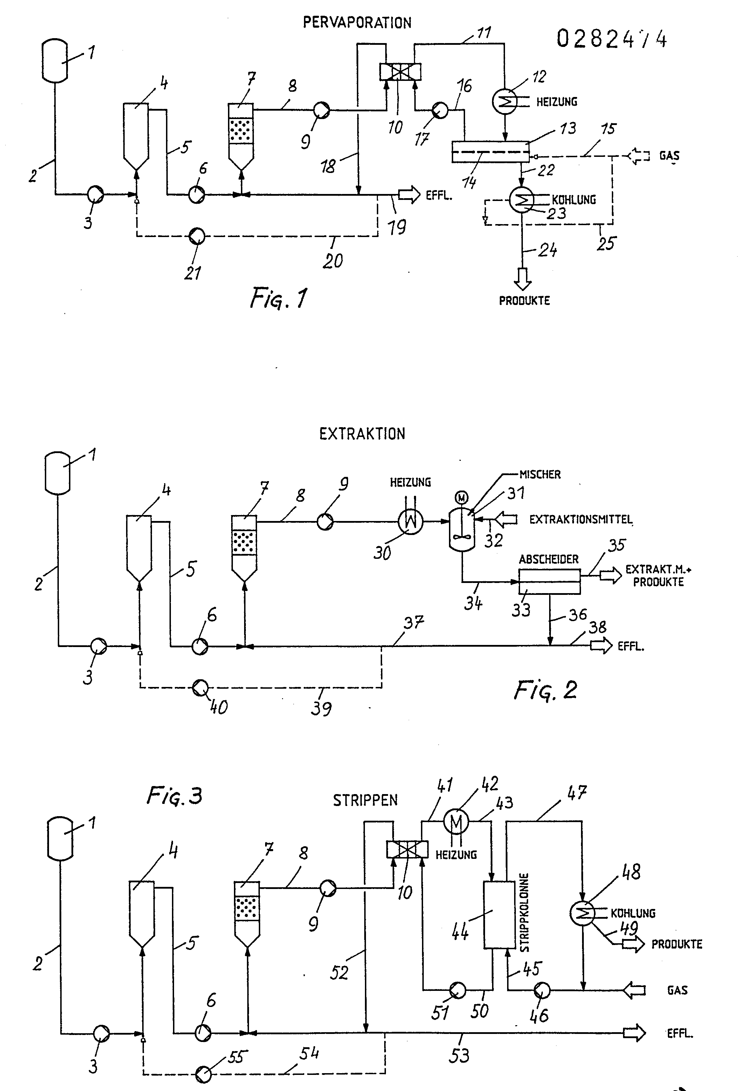

Beim Ausführungsbeispiel gemäß Fig. 1 wird das an Produkt angereicherte Medium über einen Wärmetauscher 10 geführt, in welchem es gegen rückströmendes, vom Produkt bereits befreitem Medium aufgewärmt wird. Das aufgewärmte, an Produkt reiche Medium wird über die Leitung 11 einer Heizung 12 zugeführt und von dort einem Pervaporationsmodul 13 zugeleitet. In diesem Pervaporationsmodul 13 befindet sich eine Membran 14, an deren einer Seite Medium und an deren anderer Seite ein Trägergas geführt wird. An dieser Membran wird ein Produktaustausch vom Medium zum Gas vorgenommen, wobei das Gas über eine Leitung 15 in das Pervaporationsmodul 13 eingebracht wird. Das vom Produkt befreite Medium wird über die Leitung 16 und eine Pumpe 17 dem Wärmetauscher 10 zugeführt und über die Leitung 18 wieder in den Festbettreaktor rückgeführt. Überschüssiges vom Produkt weitgehend befreites Medium wird über die Leitung 19 abgeführt. Ein Teil des Mediums wird, wie in Fig. 1 strichliert eingezeichnet, über eine Leitung 20 und eine Pumpe 21 dem Rührkesselbioreaktor zugeführt. Das an Produkt angereicherte Gas wird aus dem Pervorationsmudul 13 über die Leitung 22 einer Kühleinrichtung 23 zugeführt, in welcher das gebildete Produkt kondensiert und über die Leitung 24 abgezogen wird. Das vom Produkt befreite Gas wird über die Leitung 25 wieder dem Pervaporationsmodul 13 rückgeführt.

Beim Ausführungsbeispiel gemäß Fig. 2 wird das über die Leitung 8 und die Pumpe 9 aus dem Festbettreaktor abgezogene mit Produkt angereicherte Medium einer Heizeinrichtung 30 zugeführt, von welcher es in einen Mischer 31 gelangt. In diesen Mischer wird über die Leitung 32 Extraktionsmittel eingeleitet und das Gemisch aus Extraktionsmittel und an Produkt angereichertem Medium einem Abscheider 33 über die Leitung 34 zugeleitet. Im Abscheider 33 werden dann das mit Produkten beladene Extraktionsmittel und das vom Produkt befreite medium voneinander getrennt, wobei das mit Produkten angereicherte Extraktionsmittel über die Leitung 35 und das vom Produkt befreite Medium über die Leitung 36 abgezogen wird. Das vom Produkt befreite Medium wird dann über die Leitung 37 in den Festbettreaktor rückgeführt, überschüssiges Medium wird über die Leitung 38 abgezogen. Wie in Fig.2 strichliert angedeutet, kann auch vom Produkt befreites Medium über eine Leitung 39 und eine Pumpe 40 in den ersten Verfahrensabschnitt rückgeführt werden. Die Trennung zwischen Extraktionsmittel und Produkt kann in herkömmlicher Weiser durch Destillation u.dgl. vorgenommen werden.

Beim Ausführungsbeispiel gemäß Fig. 3 wird das über die Leitung 8 und die Pume 9 abgezogene, an Produkt angereicherte Medium wieder einem Wärmetauscher 10 zugeführt, von welchem es über die Leitung 41 einer Heizung 42 zugeführt wird, von wo es über die Leitung 43 in eine Strippkolonne 44 gelangt. In dieser Strippkolonne wird das an Produkt angereicherte Medium im Gegenstrom mit Gas behandelt, welches über die Leitung 45 und die Pumpe 46 in die Kolonne eingeblasen wird. Das an Produkt angereicherte Strippgas wird über die Leitung 47 einere Kühleinrichtung 48 zugeführt, in welcher die Produkte kondensieren und über die Leitung 49 abgezogen werden. Das vom Produkt freie Gas wird wieder über die Pumpe 46 und die Leitung 47 in die Strippkolonne 44 rückgeführt. Das vom Produkt befreite Medium wird über die Leitung 50 und die Pumpe 51 dem Wärmetauscher 10 zugeführt, in welchem das noch warme Medium gegen frisches, vom Festbettreaktor 7 kommenden Medium geführt wird. Vom Wärmetauscher 10 gelangt das vom Produkt befreite Medium über die Leitung 52 in den Festbettreaktor 7. Auch in vorliegendem Fall wird überschüssiges Medium über eine Leitung 53 aus dem System abgezogen. Die strichliert eingezeichnete Leitung 54 und die Pumpe 55 dienen wieder zur Rückführung von vom Produkt befreiten Medium in den ersten Verfahrensabschnitt, nämlich in den Rührkesselbioreaktor 4.

Das erfindungsgemäße Verfahren wird nachstehend an Hand von Ausführungsbeispielen näher erläutert.

Beispiel 1:

Als Nährmedium wird ein Medium folgender Zusammensetzung genommen:

Xylose (wasserfrei) 60g/l
$KH_2PO_4$     1g/l
$K_2HPO_4$     1g/l
$(NH_4)_2SO_4$     2g/l
$MgSO_4.7H_2O$     0,1g/l
NaCl     0,01g/l
$Na_2MoOO_4.2H_2O$     0,01g/l
$CaCl_2$     0,01g/l
$MnSO_4.H_2O$     0.015g/l
$FeSO_4.7H_2O$     0.015g/l
Biotin     0,1mg/l
Thiaminiumchlorid     0,002g/l
p-Aminobenzoesäure     0,002g/l
$Na_2S_2O_4$     0,035g/l
Resazurin     0,001g/l
Hefeextrakt     0,5g/l
$Na_4OOCCH_3$     2,2g/l.

Als Gärungsorganismus wird Clostridium acetobutylicum ATCC 824 eingesetzt. Der Gärungsorganismus wird in entsprechender Vorkultur herangezogen, bevor er in das erfindungsgemäße Verfahren eingeführt wird.

Das Nährmedium wird aus dem Vorratsbehälter 1 kommend in den Rührkesselbioreaktor 4 eingeleitet, u.zw. mit einer Durchflußrate von $0.3xh^{-1}$. Das Inokulum beträgt dabei etwa 10% des Inhalts des Rührkesselbioreaktors 4. Die Fermentation wird im Rührkesselbioreaktor bei einer Temperatur von $37°C$ geführt, wobei innerhalb des Reaktors ein pH von 5,1 gehalten wird. Durch die gewählte Durchflußrate wird die Zelldichte innerhalf des Bioreaktors 4 etwa gleich gehalten, wobei die auf Grund des Bakterienwachstums entstehenden neuen Zellen über die Leitung 5 in den Festbettreaktor 7 eingebracht werden, wo sie durch Adhäsion an dem Trägermaterial haften bleiben. Als Trägermaterial wird offenporiges Glassintermaterial verwendet, wobei das Volumen des Glassintermateriales 50% des Gesamtarbeitsvolumens beträgt. Im Festbettreaktor 7 wird eine Fermentationstemperatur von 33°C eingehalten, wobei die Fermentation bei einem pH von 4,3 ausgeführt wird. Die Gesamtdurchflußrate nach beiden Verfahrensabschnitten beträgt 0,15 x h⁻¹. Innerhalb des Festbettreaktors 7 wird der Reaktorinhalt mittels Stickstoff begast, wodurch zufolge von Leiteinrichtungen, welche im Festbettreaktor angeordnet sind, eine Zirkulation erzielt wird.

Die Produktivität und die Ausbeute werden nachstehend in Tabelle 1 zusammengefaßt.

## Tabelle 1

| | Rest - Xylose [g/l] | Zuk= ker- Ab= bau [%] | Zuk= ker- nut= zung [g/l.h] | Bu- ta- nol [g/l] | Ge= saat- LM [g/l] | LM- Aus- beu= te [%] | LM- Pro= duk= tivi tät [g/lh] | Ge= saat Säu= ren [g/l] | Säuren- Aus= beute [%] | Opt. Dich= te | pH- Wert | Eh- Wert [mV] |
|---|---|---|---|---|---|---|---|---|---|---|---|---|
| D = 0.15 h-1 | 28.59 | 50.98 | 4.46 | 5.7 | 9.30 | 31.28 | 1.395 | 3.06 | 5.88 | 2.765 | 4.32 | -436 |

LM = Lösungsmittel

Eh = Potentialmessung

Beispiel 2

Es wird die gleiche Nährstoffzusammensetzung und das gleiche Inokulum und auch die gleichen Verfahrensbedingungen innerhalb des Rührkesselbioreaktors und des Festbettreaktors gewählt. Am Festbettreaktor wird eine externe Schlaufe angeschlossen, in welcher die gebildeten Produkte kontinuierlich oder chargenweise abgezogen werden. Dazu wird die vom Festbettreaktor abgezogene, mit Produkt angereicherte Flüssigkeit auf eine Temperatur von etwa 40° C aufgeheizt und einer Strippkolonne zugeführt, in welcher der Flüssigkeit Stickstoff als Strippgas im Gegenstrom geführt wird. Das Verhältnis der Volumenströme von Flüssigkeit und Gas beträgt 0,35:1. Das von Produkten weitgehend befreite flüssige Medium wird nach Abkühlung auf Gärtemperatur zum Teil wieder in den Festbettreaktor 7 übergeführt, wobei ein geringerer Teil auch in den Rührkesselbioreaktor 4 rückgeführt werden kann.

Die überschüssige, vom Produkt weitgehend befreite Flüssigkeit wird aus dem System abgezogen.Das an Produkt angereicherte, von der Strippkolonne abgezogene Gas wird über eine Kühleinrichtung geführt, in welcher die Produkte kondensieren und abgezogen werden können. Die erzielten steady state-Werte sind nachstehend in Tabelle 2 zusammengefaßt.

## Tabelle 2

| ! Rest - ! | Zuk= ! | Zuk= ! | Bu- ! | Ge= ! | LM- ! | LM- | Ge= | ! Säuren- ! | Opt. ! | pH- ! | Eh- ! |
| Xylose ! | ker- ! | ker- ! | ta- ! | saat-! | Aus- ! | Pro= | saat | ! Aus= | Dich= ! | Wert ! | Wert ! |
| ! | Ab= ! | nut= ! | nol ! | LM ! | beu= ! | duk= | Säu= | beute | te ! | ! | ! |
| ! | bau ! | zung ! | ! | ! | te ! | tivi= | ren | ! | ! | ! | ! |
| ! | ! | ! | ! | ! | ! | tät | ! | ! | ! | ! | ! |
| ! [g/l] ! | [%] ! | [g/l.h]! | [g/l]! | [g/l]! | [%] ! | [g/lh] | [g/l] ! | [%] ! | ! | ! | [mV] ! |
| 1 t 4.15 | h-1 ! 21.50 ! | 62.50 ! | 5.37 ! | 6.82 ! | 10.87 ! | 30.36! | 1.638 | ! 3.76 ! | 6.59 ! | 2.41 ! 4.10 ! | -450 ! |

LM = Lösungsmittel

Eh = Potentialmessung

Wie aus den Tabellen ersichtlich, ist die Produktivität und der Zuckerabbau bei dem Verfahren gemäß Beispiel 2, also bei kontinuierlicher Produktentnahme in einer externen Schlaufe deutlich besser. Dieses Ergebnis zeigt sich auch dann, wenn, wie die vorstehenden Versuche zeigten, die Gärung über einen längeren Zeitraum, vorstehend über 500 Stunden, geführt wurde.

## Patentansprüche

1. Verfahren zur Vergärung von kohlenhydrathältigen Medien mit Hilfe von Butanol, Aceton, Ethanol und/oder Isopropanol bildenden Bakterien, wobei das Verfahren wenigstens zweistufig ausgeführt wird, wobei im ersten Verfahrensabschnitt im wesentlichen die Bakterien kontinuierlich kultiviert und im zweiten, kontinuierlich oder chargenweise geführten Verfahrensabschnitt im wesentlichen das Produkt gebildet wird, dadurch gekennzeichnet, daß die Bakterien im zweiten Verfahrensabschnitt an einem Trägermaterial immobilisiert werden.

2. Verfahren nach Anspruch 1, dadurch gekennzeichnet, daß als Trägermaterial offenporiges Sinterglas, Bimsstein oder ein gleichwertiges Material, welches einen hohen $SiO_2$ Gehalt, eine offenporige Struktur, eine Porengröße von 20 - 200µm, vorzugsweise 50 - 100µm, und eine Wasseraufnahmefähigkeit von wenigstens 0,35 ml/cm³ aufweist, eingesetzt wird, und wobei das Verhältnis des Volumens des Trägermaterials zum Gesamtarbeitsvolumen 1:10 bis 1:1,1, vorzugsweise 1:5 bis 1:1,6, beträgt.

3. Verfahren nach Anspruch 1 oder 2 dadurch gekennzeichnet, daß aus der Produktbildungsstufe Medium abgezogen, das gebildete Produkt extraktiv, vorzugsweise in an sich bekannter Weise mittels höherer Alkohole oder höherer Fettsäuren, abgetrennt und die dadurch anfallende produktarme Phase in die Produktbildungsstufe und/oder den ersten Verfahrensabschnitt rückgeführt wird.

4. Verfahren nach Anspruch 1 oder 2, dadurch gekennzeichnet, daß im zweiten Abschnitt Produkt, vorzugsweise kontinuierlich, pervaporativ aus dem Medium abgeführt wird.

5. Verfahren nach Anspruch 4, dadurch gekennzeichnet, daß für die pervaporative Produktentnahme eine Pervaporationsmembran, insbesondere aus Silikonkautschuk, eingesetzt wird, wobei eine Temperatur von 30°-90°C, vorzugsweise 40°-80°C, eingehalten wird, und wobei die Dampfdruckerniedrigung pervaporationsseitig durch ein Trägergas, wie $N_2$, $H_2$, $CO_2$ oder eine Mischung dieser Gase, oder durch Fermentationsgas bzw. durch Anlegen eines Vakuums erzielt wird.

6. Verfahren nach einem der Ansprüche 1 bis 3, dadurch gekennzeichnet, daß im zweiten Abschnitt eine, vorzugsweise kontinuierliche, Produktabtrennung mittels Gasstrippen durchgeführt wird.

7. Verfahren nach Anspruch 6, dadurch gekennzeichnet, daß für die kontinuierliche Produktabtrennung durch Gasstrippen ein Strippgas, wie $N_2$, $H_2$, $CO_2$ oder Mischungen dieser Gase, bzw. Fermentationsgas eingesetzt wird, wobei das Verhältnis der Volumensströme von Flüssigkeit zu Gas 0,05:1 - 1,6:1 und die Temperatur 30° - 90°C, vorzugsweise 40° - 80°C, beträgt.

8. Verfahren nach einem der Ansprüche 1 bis 4, dadurch gekennzeichnet, daß der Zulauf an Nährlösung und/oder Zuckerlösung in Abhängigkeit vom Produktgehalt des zweiten Verfahrensabschnittes geregelt wird.

9. Vorrichtung zur Durchführung des Verfahrens nach einem der Ansprüche 1 bis 9, dadurch gekennzeichnet, daß ein Bioreaktor, vorzugsweise ein Rührkesselbioreaktor (4), und ein mit Mikroorganismen rückhaltendem Material bestückter Reaktor, vorzugsweise ein Festbettreaktor (7), hintereinandergeschaltet sind, wobei gegebenenfalls an den Festbettreaktor (7) eine externe Schlaufe zur, vorzugsweise kontinuierlichen, Produktabtrennung angeschlossen ist.

10. Vorrichtung nach Anspruch 9, dadurch gekennziechnet, daß innerhalb des Festbettreaktors ein oder mehrere Leiteinrichtungen zur Erzielung einer Zirkulation innerhalb des Reaktors vorgesehen sind.

11. Vorrichtung nach Anspruch 10, dadurch gekennzeichnet, daß zur Erzielung der Zirkulation örtlich Gaseinbringeinrichtungen oder Leitungen zum gerichteten Einströmen von, vorzugsweise rückgeführtem, Medium vorgesehen sind.

12. Vorrichtung nach Anspruch 9, dadurch gekennzeichnet, daß in der externen Schlaufe zur Produktabtrennung ein Pervaporationsmodul (13) angeordnet ist.

13. Vorrichtung nach Anspruch 12, dadurch gekennzeichnet, daß in dem Pervaporationsmodul (13) eine Membran (14) insbesondere eine Silikonkautschukmembran, vorgesehen ist.

14. Vorrichtung nach Anspruch 9, dadurch gekennzeichnet, daß in der externen Schlaufe zur Produktabtrennung eine Gegenstromstrippkolonne (44) angeordnet ist.

PERVAPORATION

0282474

HEIZUNG

GAS

KÜHLUNG

EFFL.

PRODUKTE

Fig. 1

EXTRAKTION

HEIZUNG

MISCHER

EXTRAKTIONSMITTEL

ABSCHEIDER

EXTRAKT.M.+ PRODUKTE

EFFL.

Fig. 2

Fig. 3

STRIPPEN

HEIZUNG

STRIPPKOLONNE

KÜHLUNG

PRODUKTE

GAS

EFFL.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | BIOTECHNOLOGY & BIOENGINEERING, Band 26, Nr. 8, August 1984, Seiten 992-997, John Wiley & Sons, Inc., New York, US; M. NAGASHIMA et al.: "Continuous ethanol fermentation using immobilized yeast cells" * Seite 992, linke Spalte, Absatz 1; Seite 993, linke Spalte, Absätze 4,5; Seite 994, Figur 7 * --- | 1,9-11 | C 12 P 7/06 C 12 P 7/16 C 12 P 7/26 |
| X | CHEMICAL ABSTRACTS, Band 100, Nr. 25, 18. Juni 1984, Seite 443, Zusammenfassung Nr. 207895z, Columbus, Ohio, US; & JP-A-59 45 891 (MITSUBISHI KAKOKI KAISHA, LTD) 14-03-1984 * Zusammenfassung * --- | 1 | |
| Y | GB-A-2 113 711 (HITACHI) * Ansprüche 1,2,4; Beispiel 1; Figur 1 * --- | 1,2,9 | |
| Y | EP-A-0 112 459 (CPC INTERNATIONAL) * Ansprüche 1,3; Beispiel 1 * --- | 1,2,9 | RECHERCHIERTE SACHGEBIETE (Int. Cl.4) C 12 P |
| A | BIOTECHNOLOGY & BIOENGINEERING SYMPOSIUM, Nr. 17, 1986, Seiten 519-533, John Wiley & Sons, Inc., New York, US; C. SOLA et al.: "Continuous ethanol production by immobilized yeast cells and ethanol recovery by liquid-liquid extraction" * Seite 519, Zusammenfassung * --- -/- | 3 | |

Der vorliegende Recherchenbericht wurde für alle Patentansprüche erstellt

| Recherchenort | Abschlußdatum der Recherche | Prüfer |
|---|---|---|
| DEN HAAG | 07-06-1988 | WIESER, M.R.J. |

**Europäisches Patentamt**

# EUROPÄISCHER RECHERCHENBERICHT

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | CHEMICAL ABSTRACTS, Band 102, Nr. 13, 1. April 1985, Seite 544, Zusammenfassung Nr. 111418m, Columbus, Ohio, US; W.J. GROOT et al.: "Increase of substrate conversion by pervaporation in the continuous butanol fermentation", & BIOTECHNOL. LETT. 1984, 6(12), 789-92 * Zusammenfassung * --- | 4,5,12, 13 | |
| A | GB-A-2 054 645 (ALFA-LAVAL) * Anspruch 1; Beispiel 1; Figur 1 * ----- | 6,7,14 | |
| | | | RECHERCHIERTE SACHGEBIETE (Int. Cl.4) |

Der vorliegende Recherchenbericht wurde für alle Patentansprüche erstellt

| Recherchenort | Abschlußdatum der Recherche | Prüfer |
|---|---|---|
| DEN HAAG | 07-06-1988 | WIESER, M.R.J. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
...........................................................................................
& : Mitglied der gleichen Patentfamilie, übereinstimmendes Dokument

EPO FORM 1503 03.82 (P0403)